(19) Europäisches Patentamt
European Patent Office
Office européen des brevets

(11) Veröffentlichungsnummer: **0 204 736 B1**

(12) # EUROPÄISCHE PATENTSCHRIFT

(45) Veröffentlichungstag der Patentschrift: **26.07.89**

(51) Int. Cl.⁴: **C 07 C 47/228**, C 07 C 45/67, C 07 C 45/59, C 07 C 59/64, C 07 C 69/738, C 07 C 69/734, C 07 D 317/30

(21) Anmeldenummer: **85905753.1**

(22) Anmeldetag: **27.11.85**

(86) Internationale Anmeldenummer: **PCT/CH 85/00168**

(87) Internationale Veröffentlichungsnummer: **WO 86/03486 (19.06.86** Gazette **86/13)**

(54) **VERFAHREN ZUR HERSTELLUNG VON BENZOLDERIVATEN.**

(30) Priorität: **05.12.84 CH 5781/84**
**13.11.85 CH 4866/85**

(43) Veröffentlichungstag der Anmeldung: **17.12.86 Patentblatt 86/51**

(45) Bekanntmachung des Hinweises auf die Patenterteilung: **26.07.89 Patentblatt 89/30**

(84) Benannte Vertragsstaaten: **AT BE CH DE FR GB IT LI NL**

(56) Entgegenhaltungen:
**DE-A- 2 628 469**
**FR-A- 657 691**
**US-A- 2 976 321**

(73) Patentinhaber: **L. GIVAUDAN & CIE Société Anonyme, CH-1214 Vernier-Genève (CH)**

(72) Erfinder: **BAUDIN, Josianne, 6, rue René Naudin, F-74100 Annemasse (FR)**
Erfinder: **GONZENBACH, Hans, Ulrich, 61 bis, rue de Lyon, CH-1203 Genève (CH)**

(74) Vertreter: **Urech, Peter, Dr. et al, Grenzacherstrasse 124 Postfach 3255, CH-4002 Basel (CH)**

EP 0 204 736 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von p-tert. Butyl-benzolderivaten.

Die nach dem erfindungsgemässen Verfahren erhaltenen Produkte sind neue Zwischenprodukte, die zur Herstellung eines bekannten Riechstoffes geeignet sind, bzw. dieser Riechstoff selbst.

Das Verfahren ist dadurch gekennzeichnet, dass man p-tert. Butyl-benzylchlorid mit einem 2-Formylpropionat der Formel

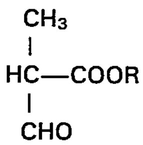
II

worin R $C_{1-6}$-Alkyl, insbesondere Methyl oder Äthyl, darstellt,
umsetzt, und die erhaltene Verbindung der Formel

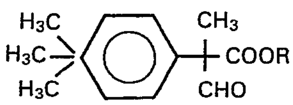
I

worin R obige Bedeutung hat,
gegebenenfalls nach Reinigung, durch Decarbalkoxylierung in p-tert. Butyl-α-methyl-hydrozimtaldehyd überführt.

p-tert. Butyl-benzylchlorid ist gemäss DT-OS 3 320 020 auf wirtschaftliche Weise in praktisch reiner Form zugänglich. Es kann nun dieses Halogenid zwar nach Sommelet zunächst zum p-tert. Butylbenzaldehyd oxydiert werden, und der letztgenannte Aldehyd mit Proprionaldehyd zum p-tert. Butyl-α-methyl-zimtaldehyd kondensiert werden. Durch Hydrierung der Doppelbindung der Seitenkette resultiert der bekannte Riechstoff p-tert. Butyl-α-methyl-hydrozimtaldehyd (US-PS 2 875 131).

Die vorliegende Reaktionsfolge ist rationeller, sie vermeidet zunächst die Sommelet-Reaktion, die infolge Anfall von N-Verbindungen, wie Methylamin und Ammoniak, zu einer unerwünschten Belastung der Umwelt mit Schadstoffen führt; zusätzlich wird die energetisch ungünstige Reihenfolge Oxidation / (Kondensation) / Reduktion vermieden.

Ein weiterer Vorteil resultiert dadurch, dass die Reaktionsprodukte der einzelnen Stufen in ausreichender Reinheit anfallen, so dass sie vor allfälligen weiteren, erfindungsgemässen Umsetzungen nicht gereinigt werden müssen.

Zwar wäre es einfacher und naheliegender gewesen, aus dem p-tert. Butyl-benzylchlorid

a) durch Alkylierung von Propionaldehyd direkt zum p-tert. Butyl-α-methyl-hydrozimtaldehyd zu gelangen, oder

b) durch Alkylierung von einem maskierten bzw. modifizierten Propionaldehyd (z.B. einem Enamin, einem Imin, etc.) über die maskierte bzw. modifizierte Form des p-tert. Butyl-α-methyl-hydrozimtaldehyds zum letzteren zu gelangen, aber diese zwei Wege liessen sich leider nicht realisieren.

Schliesslich wäre es auch einfacher gewesen, die Verbindung I über die freie Säure direkt in den p-tert. Butyl-α-methyl-hydrozimtaldehyd überzuführen, aber auch dieser Weg liess sich nicht realisieren.

Die Umsetzung von p-tert. Butyl-benzylchlorid mit der Verbindung der Formel II kann unter allgemein bekannten Alkylierungsbedingungen durchgeführt werden.

Man arbeitet also zweckmässigerweise in homogener Phase, und zwar in einem nur schwach polaren Lösungsmittel, z.B. einem aliphatischen oder aromatischen Kohlenwasserstoff, wie Pentan, Hexan, Heptan, Octan etc. oder Benzol, Toluol, etc. und in Anwesenheit einer nicht-nucleophilen Base. Geeignete Basen sind insbesondere Metallhydride, wie Alkalimetallhydride, z.B. Lithium-, Natrium- oder Kalihydrid, oder tertiäre Alkoholate, wie Natriumtert. amylat oder Kalium-tert. butylat, etc. Anwesenheit einer quaternären Ammoniumverbindung als Katalysator ist angezeigt, siehe z.B. Walter E. Keller, Compendium of Phase-Transfer Reaktions and Related Synthetic Methods, 1. Auflage, Fluka AG, CH-9470 Buchs, Schweiz, 1979, 33.

Die Reaktionstemperatur ist nicht kritisch, man kann z.B. bei Raumtemperatur oder bei erhöhter Temperatur arbeiten.

Die gebildeten Formylester der Formel I sind neue Verbindungen, die ebenfalls Gegenstand der vorliegenden Erfindung darstellen.

Die allfällige weitere Umsetzung des Formylesters I erfolgt erfindungsgemäss durch Decarbalkoxylierung, d.h. Entfernen der Carbonsäureestergruppe, und zwar unter Vermeidung von Decarbonylierung bzw. Deformylierung.

Diese Decarbalkoxylierung der Verbindung I kann z.B. dadurch bewerkstelligt werden, dass die Verbindung I zunächst in eine Verbindung der Formel

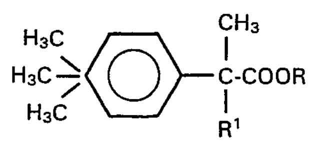
III

worin R obige Bedeutung besitzt und $R^1$ eine geschützte Formylgruppe, z.B. ein Acetal (ein Dialkoxymethylrest), z.B. Dimethylacetal, Diäthylacetal, oder ein cyclisches Derivat, wie Äthylenacetal (2,5-Dioxa-cyclopentyl), etc. darstellt,
übergeführt werden.

Zweckmässigerweise wird die Verbindung I acetalisiert.

Diese Acetalisierung kann nach der bekannten Methode der sauren Umsetzung von Aldehyden mit ein- oder zweiwertigen Alkoholen, bzw. mit Orthoameisensäurealkylestern, in möglichst wasserfreiem Medium durchgeführt werden, siehe z.B. Organikum, Organisch-Chemisches Grundpraktikum, Autorenkollektiv, 6. Auflage, VEB, Deutscher Verlag der Wissenschaft, Berlin, 1967, 376 ff.

Hierauf wird die Estergruppe von III in an sich bekannter Weise durch übliche Basen- und Hitzebehandlung verseift, wie dies für Carbonsäureester gängig ist (Organikum, loc. cit., 376, 399 ff).

Es entstehen dabei die Säuren der Formel

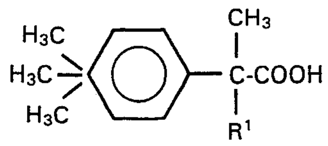

IV

worin $R^1$ eine geschützte Formylgruppe, z.B. ein Acetal (ein Dialkoxymethylrest), z.B. Dimethylacetal, Diäthylacetal, oder ein cyclisches Derivat, wie Äthylenacetal (2,5-Dioxa-cyclopentyl), etc. darstellt.

Diese Säuren sind neu und bilden einen weiteren Gegenstand der vorliegenden Erfindung.

Ihre Überführung in den p-tert. Butyl-α-methyl-hydrozimtaldehyd kann schliesslich durch eine saure, an sich bekannte Spaltung der Schutzgruppen von IV unter Hitzeeinwirkung, z.B. bei Rückflusstemperaturen, erfolgen. Bei dieser Spaltung der Schutzgruppe erfolgt zugleich Decarboxylierung, da die intermediär gebildete α-Formyl-carbonsäure

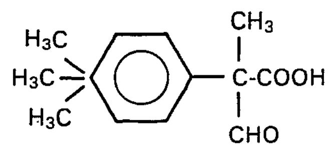

IV'

wenig stabil ist (Organikum, loc. cit. 377).

Man arbeitet zweckmässigerweise in einem Lösungsmittel, z.B. einem aliphatischen Keton, wie Aceton, Methyläthylketon, Diäthylketon, etc.

Nach einer weiteren Verfahrensvariante wird die Decarbalkoxylierung von I mittels eines nucleophilen Agens initiiert.

Das nucleophile Agens dient dazu, den Carbonsäurealkylester zu dealkylieren, also den Ester I in das Anion der Säure IV' überzuführen.

Vorzugsweise wird dabei NaI oder LiI verwendet [siehe F. Elsinger, J. Schreiber, A. Eschenmoser, Helv. Chim. Acta 43, (1960), 113 ff. und P. Müller, B. Siegfried, Tetrahedron Letters, (1973), 3565 ff.]. Im Falle von LiI kann das wasserfreie oder auch kristallwasserhaltige Material eingesetzt werden. Aber auch die anderen Alkalijodide, und auch andere Alkalihalogenide, wie die Chloride oder Bromide, oder Pseudohalogenide, z.B. Rhodanide, kommen in Betracht.

Die Menge des eingesetzten Salzes ist mindestens stöchiometrisch, doch ist natürlich auch ein Überschuss möglich. Ein höherer Überschuss als beispielsweise ca. 10-20% ist jedoch unwirtschaftlich.

Erfindungsgemäss wird dabei in einem hochsiedenden polaren Lösungsmittel, z.B. Hexamethylphosphorsäuretriamid (HMPT) gearbeitet. Dabei wird die Verbindung III zweckmässigerweise in eine heisse — z.B. 200-250°C — Lösung des Nucleophilen in HMPT getropft. Die Hitzebehandlung führt dabei gleichzeitig zu einer Decarboxylierung.

Anstelle von HMPT kann aber auch ein Polyäther, z.B. Polyäthylenoxid, Polypropylenoxid, Diglym, etc. verwendet werden.

*Beispiel 1*

a) Ein Reaktionsgefäss, das mit Rührer, Thermometer und Tropftrichter ausgerüstet ist, wird mit Stickstoff gespült und dann werden 1,9 g Natriumhydrid (55%ige Dispersion in Mineralöl, 43 mMol) mit Hexan vom Öl befreit und 50 ml trockenes Toluol zugegeben; Die Suspension wird auf 60°C erhitzt. Innert 5 Minuten werden 5 g (43,1 mMol) frisch destilliertes Methyl-α-formylproprionat in 25 ml trockenem Toluol zugegeben. Nach einer Stunde kühlt man das Gemisch auf Zimmertemperatur ab und gibt 1,5 g (3 mMol) Methyl-trioctylammoniumjodid zu. Das Methyl-trioctylammoniumjodid wurde vorgängig mit Toluol azeotrop getrocknet. Hierauf fügt man rasch 7,9 g (43,1 mMol) p-tert. Butyl-benzylchlorid in 25 ml trockenem Toluol zu. Man rührt das Gemisch 8 Stunden, lässt es 12 Stunden stehen und rührt nochmals 4 Stunden bei 45°C. Eine gaschromatographische Analyse ergibt, dass die Reaktion nach dieser Zeit beendigt ist. Man gibt bei Zimmertemperatur vorsichtig Wasser zu und extrahiert das Gemisch mit Äther. Die organische Phase wird mit Wasser, 2N Schwefelsäure, 10%igem Natriumbicarbonat und gesättigter Kochsalzlösung gewaschen, über Magnesiumsulfat getrocknet und konzentriert. Es resultieren 13,9 g rohes Öl, welches noch 2,6 g Toluol enthält. Der Rest besteht gemäss gaschromatographischer Analyse zu 86% aus Methyl-2-formyl-2-p--tert. butyl-benzoyl-propionat. Die Ausbeute beträgt 86%. Eine Probe wird destilliert und zeitigt folgende Daten:

NMR (60 MHz, $CDCl_3$) [ppm (δ)]: 1,33 Singulett breit 12 H, 3,15 Doublett J = 2 Hz 2 H, 3,75 Singulett scharf 3H, 7-7,5 Multiplett 4 H, 9,9 Singulett scharf 1 H.

a bis) Auf analoge Weise erhält man durch Alkylierung von Äthyl-α-formylpropionat das Äthyl-2-formyl-2-p-tert. butyl-benzyl-propionat.

NMR (60 MHz, $CDCl_3$) [ppm (δ)]: 1,32 Singulett herausragend aus dem Signalkomplex von total 15 H, 3,15 Doublett J = 2 Hz 2 H, 4,22 Quartett J = 7 Hz 2 H, 7-7,5 Multiplett 4 H, 9,9 singulett scharf 1 H.

b) 9,8 g des obigen Formylesters (enthaltend 26 mMol Aktivsubstanz) werden in 50 ml Methanol gelöst und es wird 1 ml Acetylchlorid zugegeben. Man rührt das Gemisch 15 Minuten bei Zimmertemperatur und erhitzt hierauf 5 Stunden auf 60°C. Man kühlt, gibt Wasser zu, neutralisiert das Ganze mit Natriumbicarbonat, extrahiert mit Äther (vorgängig gewaschen mit gesättigter Kochsalzlösung) und trocknet die organische Phase über Magnesiumsulfat. Nach Abdampfen des Lösungsmittels verbleiben 9 g eines rohen Öls, das gemäss gaschromatischer Analyse 5% Methyl-2-formyl-2-p-tert. butyl-benzyl-propionat und 74% Methyl-2-[formyl-dimethylacetal]--2-[p-tert. butyl-benzyl]-propionat enthält; die Ausbeute beträgt demzufolge 22 mMol (85%). Eine Probe wird destilliert und zeitigt folgende Daten:

Sdp. 246°C/0,6 mmHg; $n_D^{25}$ = 1,4925.

b bis) Auf gleiche Weise wird das Äthyl-2-formyl--2-p-tert. butyl-benzyl-propionat zum Äthyl-2-[formyl-dimethylacetal]-2-[p-tert. butyl-benzyl]-propionat acetalisiert.

Sdp. 115°C/0,1 mmHg; $n_D^{20}$ = 1,4894.

b ter) Analoge Behandlung von Äthyl-2-formyl-2--p-tert. butyl-benzyl-propionat in Äthanol statt Methanol liefert das Äthyl-2-[formyl-diäthylacetal]-2--[p-tert. butyl]-propionat.

Sdp. 150°C/0,07 mmHg; $n_D^{20}$ = 1,4809.

b quater) 3stündiges Erhitzen von Äthyl-2-formyl-2-p-tert. butyl-benzyl-propionat bei Rückflusstemperatur in einem Gemisch von Äthylenglycol und Toluol (5:1), unter Zusatz von 1% p-Toluolsulfonsäure liefert das Äthyl-2-[formyl-äthylenacetal]-2--[p-tert. butyl-benzyl]-propionat.

Sdp.150°C/0,4 mmHg $n_D^{20}$ = 1,5010.

c) 9,2 g Ätznatron in 13,8 g Wasser werden zu 7,5 g des obigen Rohmaterials (18 mMol Dimethylacetal) in 25 ml Methanol gegeben. Man hält das Gemisch 1 Stunde unter Rückflusstemperatur, kühlt das Gemisch ab, verdünnt mit Wasser und extrahiert mit Äther. Nach dem Abdampfen des Lösungsmittels verbleiben 1,8 g eines neutralen Materials. Die wässrigen Phasen werden mittels 2 N Schwefelsäure auf pH 4 gebracht und mit Äther extrahiert. Der organische Extrakt wird mit gesättigter Kochsalzlösung gewaschen, über Magnesiumsulfat getrocknet und ergibt nach Abdampfen des Lösungsmittels die 2-[Formyl-dimethylacetal]-2-[p-tert. butyl-benzyl]--propionsäure in 99%iger Reinheit; Schmelzpunkt 116-118°C; Ausbeute 92%.

c bis) Die analoge Behandlung des Äthyl-2-[formyl-dimethylacetal]-2-[p-tert. butyl-benzyl]-propionats führt zum selben Produkt.

c ter) Entsprechend wird auch das Äthyl-2-[formyl-diäthylacetal]-2-[p-tert. butyl-benzyl]-propionat in Äthanol zur 2-[Formyl-diäthylacetal]-2-[p-tert. butyl-benzyl]-propionsäure verseift.

Smp. 144-145°C.

c quater) Analog wird aus dem Äthyl-2-[formyl--äthylenacetal]-2-[p-tert. butyl-benzyl]-propionat die 2-[Formyl-äthylenacetal]-2-[p-tert. butyl-benzyl]-propionsäure erhalten.

Smp. 99-101°C.

d) Unter Stickstoffatmosphäre werden 4,45 g der rohen Säure von Beispiel 1 c) oder c bis) in einem Gemisch von 50 ml Methyläthylketon und 50 ml 2 N Schwefelsäure auf Rückflusstemperatur erhitzt und 40 Minuten bei dieser Temperatur gehalten. Man kühlt hierauf ab, gibt Wasser zu, extrahiert mit Diäthyläther, wäscht die organischen Phasen mit gesättigter Kochsalzlösung und trocknet über Magnesiumsulfat. Nach Abdampfen des Lösungsmittels erhält man 3,3 g praktisch reinen p-tert. Butyl-α-methyl-hydrozimtaldehyd; Ausbeute quantitativ. 2,8 g des Aldehyds ergeben nach Kugelrohrdestillation 2,4 g Aldehyd in einer Reinheit von > 99%.

d bis) Auf ähnliche Weise wird die 2-[Formyl-diäthylacetal]-2-[p-tert. butyl-benzyl]-propionsäure und die 2-[Formyl-äthylenacetal]-2-[p-tert. butyl--benzyl]-propionsäure in p-tert. Butyl-α-methyl-hydrozimtaldehyd übergeführt, wobei im letzteren Fall Aceton anstelle von Methyläthylketon als Lösungsmittel verwendet wird.

*Beispiel 2*

a) 5 ml Hexamethylphosphortriamid (HMPT) und 1 g LiI · $H_2O$ werden unter Stickstoffatmosphäre auf 240°C erhitzt. Man gibt 1,1 g rohes Methyl-2-formyl-2-p-tert. butyl-benzyl-propionat (82%) in 1 ml HMPT zu, rührt das Gemisch 1 Minute, giesst es auf Eis und extrahiert mit Hexan. Nach Waschen mit 2 N HCL, mit Natrumbicarbonat und Wasser trocknet man über Magnesiumsulfat und dampft das Lösungsmittel ab. Man erhält 0,9 g rohen p-tert. Butyl-α-methyl-hydrozimtaldehyd. 500 mg dieses Aldehyds ergeben nach Kugelrohrdestillation 300 mg reines Material. Die Ausbeute beträgt demnach 78,8%.

b) Falls in obigem Beispiel anstelle von LiI $H_2O$ 1 g NaI eingesetzt wird, ergibt sich ein analoges Resultat.

Die in den obigen Beispielen erwähnten gaschromatographischen Analysen werden unter Verwendung der Kolonnenfüllungen: 2% Apiezon L® auf Chromosorb G (AW/DMCS 80/100)® [säuregewaschen / mit Dimethyldichlorsilan behandelt] durchgeführt.

**Patentansprüche**

1. Verfahren zur Herstellung von p-tert. Butylbenzolderivaten, dadurch gekennzeichnet, dass man p-tert. Butyl-benzylchlorid mit einem 2-Formylpropionat der Formel

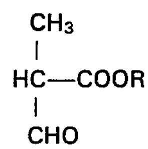

II

worin R $C_1$-$C_6$-Alkyl darstellt,
unter an sich bekannten Alkylierungsbedingungen umsetzt, und gegebenenfalls, die erhaltene Verbindung der Formel

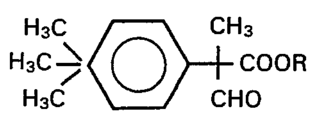

I

worin R obige Bedeutung hat,
gegebenenfalls nach Reinigung, durch Decarbalkoxylierung in p-tert. Butyl-α-methyl-hydrozimtaldehyd überführt.

2. Verfahren nach Anspruch 1, dadurch gekennzeichnet, dass man die Verbindung I unter intermediärem Schutz der Formylgruppe decarboxyliert.

3. Verfahren nach Anspruch 2, dadurch gekennzeichnet, dass man die Formylgruppe acetalisiert.

4. Verfahren nach Anspruch 1, dadurch gekennzeichnet, dass man die Decarbalkoxylierung mittels eines nucleophilen Agens in einem hochsiedenden polaren Lösungsmittel einleitet.

5. Verfahren nach Anspruch 4, dadurch gekennzeichnet, dass man als nucleophiles Agens NaI oder LiI verwendet.

6. Verbindungen der Formel

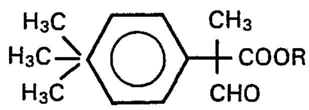 I

worin R $C_1$-$C_6$-Alkyl darstellt.

7. Methyl-2-formyl-2-p-tert. butyl-benzyl-propionat.

8. Äthyl-2-formyl-2-p-tert. butyl-benzyl-propionat.

9. Verbindungen der Formel

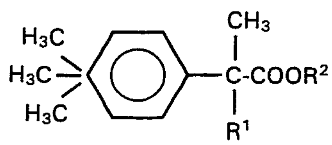 III'

worin $R_1$ eine geschützte Formylgruppe und $R_2$ Wasserstoff oder $C_1$-$C_6$-Alkyl darstellen.

10. Verbindungen gemäss Anspruch 9, dadurch gekennzeichnet, dass die Formylgruppe acetalisiert ist.

11. Methyl-2-[formyl-dimethylacetal]-2-[p-tert. butyl-benzyl]-propionat.

12. Äthyl-2-[formyl-dimethylacetal]-2-[p-tert. butyl-benzyl]-propionat.

13. Äthyl-2-[formyl-diäthylacetal]-2-[p-tert. butyl-benzyl]-propionat.

14. Äthyl-2-[formyl-äthylenacetal]-2-[butyl-benzyl]-propionat.

15. 2-[Formyl-dimethylacetal]-2-[p-tert. butyl-benzyl]-propionsäure.

16. 2-[Formyl-diäthylacetal]-2-[p-tert. butyl-benzyl]-propionsäure.

17. 2-[Formyl-äthylenacetal]-2-[p-tert. butyl-benzyl]-propionsäure.

## Claims

1. A process for the manufature of p-tert.butyl-benzene derivatives, characterized by reacting p-tert.butyl-benzyl chloride with a 2-formylpropionate of the formula

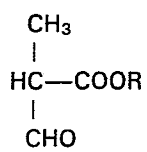 II

wherein R represents $C_{1-6}$-alkyl,
under alkylating conditions known per se and, if desired, converting the resulting component of the formula

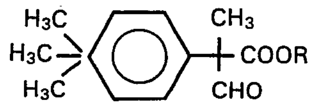 I

wherein R has the above significance,
optionally after purification, into p-tert.butyl-α-methyl-hydrocinnamaldehyde by decarbalkoxylation.

2. A process according to claim 1, characterized in that the compound I is decarbalkoxylated with the intermediary protection of the formyl group.

3. A process according to claim 2, characterized in that the formyl group is acetalized.

4. A process according to claim 1, characterized in that the decarbalkoxylation is initiated by means of a nucleophilic agent in a high-boiling polar solvent.

5. A process according to claim 4, characterized in that NaI or LiI is used as the nucleophilic agent.

6. Compounds of the formula

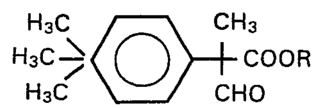 I

wherein R represents $C_1$-$C_6$-alkyl.

7. Methyl 2-formyl-2-p-tert.butyl-benzyl-propionate.

8. Ethyl 2-formyl-2-p-tert.butyl-benzyl-propionate.

9. Compounds of the formula

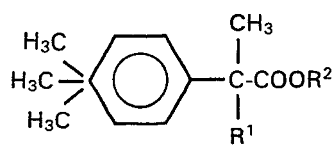 III'

wherein $R^1$ represents a protected formyl group and $R_2$ represents hydrogen or $C_{1-6}$-alkyl.

10. Compounds in accordance with claim 9, characterized in that the formyl group is acetalized.

11. Methyl 2-[formyl dimethyl acetal]-2-[p-tert.-butyl-benzyl]-propionate.

12. Ethyl 2-[formyl dimethyl acetal]-2-[-p-tert.-butyl-benzyl]-propionate.

13. Ethyl 2-[formyl diethyl acetal]-2-[p-tert.butyl-benzyl]-propionate.

14. Ethyl 2-[formyl ethylene acetal]-2-[-p-tert.-butyl-benzyl]-propionate.

15. 2-[Formyl dimethyl acetal]-2-[p-tert.butyl-benzyl]-propionic acid.

16. 2-[Formyl diethyl acetal]-2-[p-tert.butyl-benzyl]-propionic acid.

17. 2-[Formyl ethylene acetal]-2-[p-tert.butyl-benzyl]-propionic acid.

## Revendications

1. Procédé de préparation de dérivés du p-tert.-butylbenzène, caractérisé en ce que l'on fait réagir

le chlorure de p-tert.-butylbenzyle avec un 2-formylpropionate de formule:

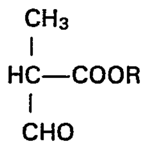

II

dans laquelle R représente un groupe alkyle en $C_1$-$C_6$, dans des conditions d'alkylation connues en soi, et, le cas échéant, on convertit le composé obtenu de formule:

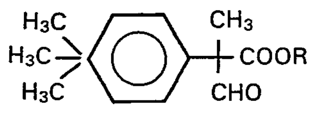

I

dans laquelle R a les significations indiquées ci-dessus, éventuellement après purification, par décarbalcoxylation, en aldéhyde p-tert.-butyl-α-méthylhydrocinnamique.

2. Procédé selon la revendication 1, caractérisé en ce que l'on soumet le composé I à décarbalcoxylation avec protection intermédiaire du groupe formyle.

3. Procédé selon la revendication 2, caractérisé en ce que l'on acétalise le groupe formyle.

4. Procédé selon la revendication 1, caractérisé en ce que l'on déclenche la décarbalcoxylation à l'aide d'un agent nucléophile dans un solvant polaire à haut point d'ébullition.

5. Procédé selon la revendication 4, caractérisé en ce que l'on utilise en tant qu'agent nucléophile NaI ou LiI.

6. Composé de formule:

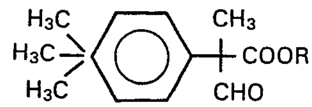

I

dans laquelle R représente un groupe alkyle en $C_1$-$C_6$.

7. Le 2 formyl-2-p-tert.-butylbenzylpropionate de méthyle.

8. Le 2-formyl-2-p-tert.-butylbenzylpropionate d'éthyle.

9. Composé de formule:

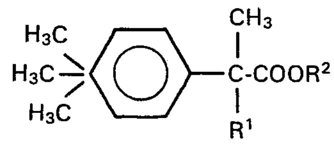

III'

dans laquelle $R^1$ représente un groupe formyle protégé et $R_2$ représente l'hydrogène ou un groupe alkyle en $C_1$-$C_6$.

10. Composé selon la revendication 9, caractérisée en ce que le groupe formyle est acétalisé.

11. Le 2-[formyldiméthylacétal]-2-[p-tert.-butylbenzyl]-propionate de méthyle.

12. Le 2-[formyldiméthylacétal]-2-[p-tert.-butylbenzyl]-propionate d'éthyle.

13. Le 2-[formyldiéthylacétal]-2-[p-tert.-butylbenzyl]-propionate d'éthyle.

14. Le 2-[formyléthylène-acétal]-2-[p-tert.-butylbenzyl]-propionate d'éthyle.

15. L'acide 2-[formyldiméthylacétal]-2-[p-tert.--butylbenzyl]-propionique.

16. L'acide 2-[formyldiethylacétal]-2-[p-tert.-butylbenzyl]-propionique.

17. L'acide 2-[formyléthylène-acétal]-2-[p-tert.--butylbenzyl]-propionique.